# EUROPEAN PATENT APPLICATION

(11) **EP 2 915 553 A1**
(43) Date of publication of application: **09.09.2015**
(21) Application number: 13851967.3
(22) Date of filing: 28.01.2013
(51) Int. Cl.: A61M 5/32

(54) **SAFE INJECTION NEEDLE**

(30) Priority: 30.10.2012 CN 201210423669
(71) Applicant: Zhang, Linfeng, Wenzhou, Zhejiang 325024 (CN)
(72) Inventor: Zhang, Linfeng, Wenzhou, Zhejiang 325024 (CN)
(74) Representative: Pulieri, Gianluca Antonio
(86) International application number: PCT/CN2013/000086
(87) International publication number: WO 2014/067221

(57) **Abstract**

The present application discloses a safety injection needle, comprising a needle base (1) provided on the end potion thereof with a needle tube (2), characterized in that the needle base (1) being provided at one side with a safety sheath (3), an elastic telescopic plate (4) being provided at the junction between the safety sheath (3) and the needle base (1), the safety sheath 3 rotatable about the elastic telescopic plate (4) which can be seen as a central supporting point, the safety sheath (3) having a hollow cavity which is provided therein in sequence with a sheath safety hook (301) which is located adjacently to the elastic telescopic plate (4) and a needle tube safety hook (302), the needle base (1) being provided with a needle base safety hook (101) cooperating with the sheath safety hook (301). The safety protection mechanism is integral with the needle base and the multiple-segment engagement mechanism, in particular reversed double hook mechanism ensure that the safety protection mechanism cannot separate from the needle tube after they engage with each other so that a safety factor of the injection needle is improved efficiently and the whole design of the product is reasonable and the conceivability is out of box and the operation of it is convenient and efficient. Compared to the conventional product, the present invention possesses prominent substantive feature and notable development.

## Description

### Technical field

The present invention relates to a medical instrument technical field, and in particular, to a safety injection needle which is provided with an overturning sheath, especially to a mechanical connection structure of the safety injection needle.

### Background art

Cited document 1: Patent no.ZLCN201120205360. 7; application date: June16,2011; publication no. CN202096549U; patentee: Chengdu Pushen Pharmaceutical Plastic Packaging Co., LTD, disclosing a safety injection needle and the independent claim of which defines as follows: comprising a needle base used for fixing the needle tube, and a sheath removeably installed on the needle base, characterized in that the needle base is provided with a guide sleeve extending upward in a longitudinal direction, which the guide sleeve is provided on the upper portion with a sliding sleeve which is provided on the top end with a needle passage. A locking structure is provided between the sliding sleeve and the guide sleeve; the guide sleeve is provided therein with a groove containing a compression spring to be applied to the bottom of the sliding sleeve which is provided therein with an elastic stop plate capable of blocking the needle passage.

It is seen from the prior art that the safety structure of the conventional safety injection needle adopts split-typed structure which is quite complicated. Operational errors occur easily when being utilized, so it is necessary to further improve the conventional structure of the safety injection needle.

### Summary of the invention

The present invention has a purpose of proposing a safety injection needle in which a safety protection structure is integral with the needle base body, which can be operated conveniently and fast, and with a stable working state and a high safety factor, and thereby overcomes the disadvantage and deficiency in the prior art.

To achieve the above purpose, a technical solution of the present invention lies in that: a safety injection needle, comprising a needle base with a needle tube on the end portion thereof, characterized in that on one side of the said needle base is provided a safety sheath. At the junction between the safety sheath and the needle base is provided an elastic telescopic plate which can be regarded as a central supporting point about which the safety sheath is rotatable; the safety sheath has a hollow cavity in which are provided in sequence a sheath safety hook which is located adjacently to the elastic telescopic plate and a tube safety hook; a needle base safety hook is provided at the place of the needle base and is used for cooperating with the sheath safety hook.

The present invention discloses a safety injection needle whose safety protection structure is integral with the needle base body. a multiple-segment engagement structure, especially a structure of reversed double hooks can ensure the safety protection structure and the needle tube can not separate after they engage with each other, and thus the safety factor of the injection needle is highly improved, with a reasonable and ingenious design of the safety injection needle, and a convenient and efficient operation. Comparing to the prior art, the present invention has outstanding substantive features and notable effects.

### Descriptions of the drawings

Fig.1 is a schematic view of the structure of the present invention;
Fig.2 is a schematic view of the structure of the safety sheath of the present invention;
Fig3. is a schematic view of the structure the sheath safety hook of the present invention;
Fig4. is a schematic view of the structure of the needle tube safety hook of the present invention;
Fig.5 is a first functional state of the present invention;
Fig.6 is a second functional state of the present invention;
Fig.7 is a third functional state of the present invention;
Fig.8 is a fourth functional state of the present invention;
Fig.9 is a fifth functional state of the present invention;
Fig. 10 is a sixth functional state of the present invention;
Fig. 11 is a seventh functional state of the present invention;

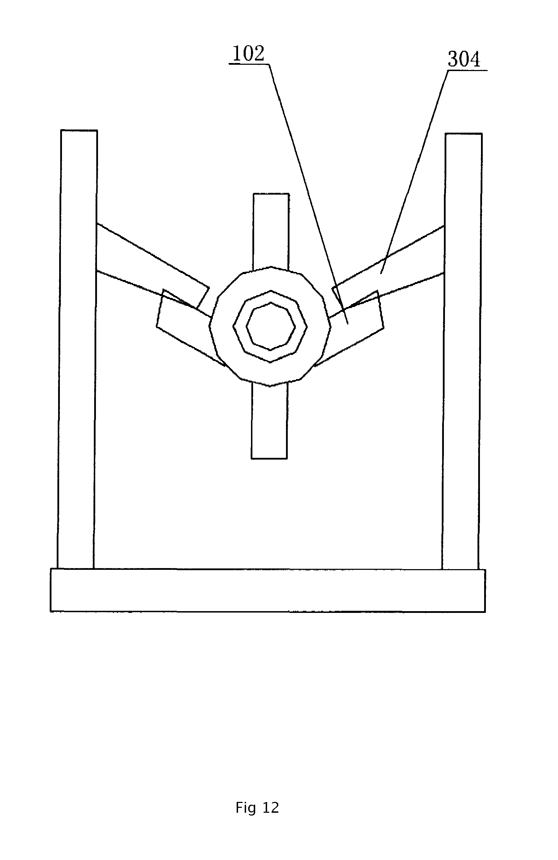

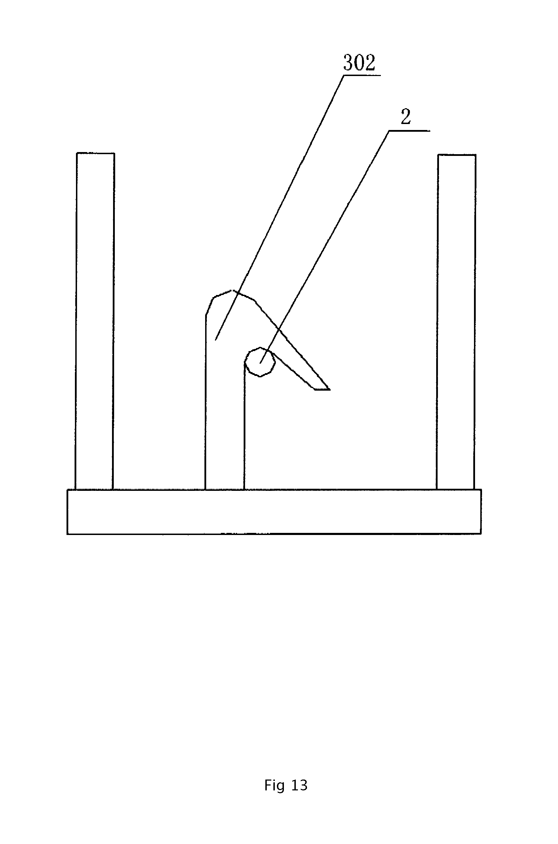

Fig. 12 is a schematic view of engagement of the sheath safety hook and the needle safety hook; and

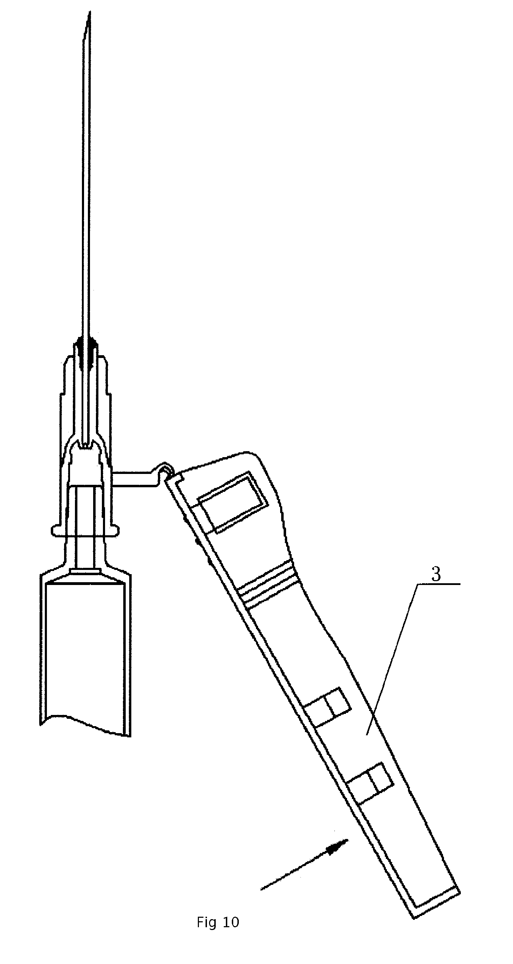

Fig. 10 is a schematic view of engagement of the needle tube safety hook and the needle tube.

### Best carried-out examples

Referring to the drawings, the present invention is more detailed described.

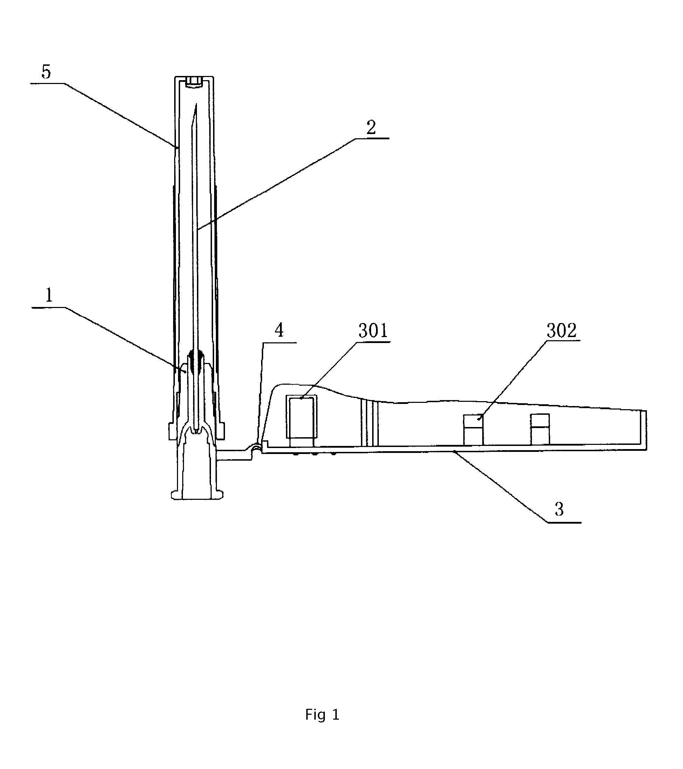

The present invention relates to a safety injection needle as shown in Fig.1. It comprises a needle base 1 which is provided on the end portion with a needle tube 2, and on the outer portion thereof with a sheath 5. The present invention distinguishes from the prior art in that the needle base 1 is provided at one side with a safety sheath 3, and an elastic telescopic plate 4 where an arc upheaval is formed is provided at the junction between the safety sheath 3 and the needle base 1; the safety sheath 3 rotates about the elastic telescopic plate 4 which can seen as a central supporting point, and has a hollow cavity in which are provided in sequence a sheath safety hook 301 which is located adjacently to the elastic telescopic plate 4 and a tube safety hook 302. The needle base 1 is provided with a needle base safety hook 101 cooperating with the sheath safety hook 301.

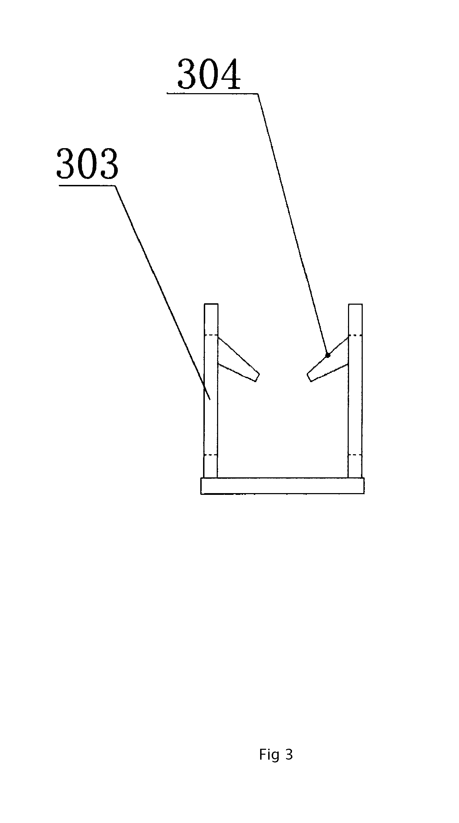

When the invention is carried out in detail, as shown in fig.3, the safety sheath 3 is provided on the surface with openings, on the two sides with protective wings 303. The sheath safety hook 301 is in form of two reversed hooked teeth 304 which are placed symmetrically and whose tooth roots are provided on the inner wall of protective wings and whose tooth ends are in the air.

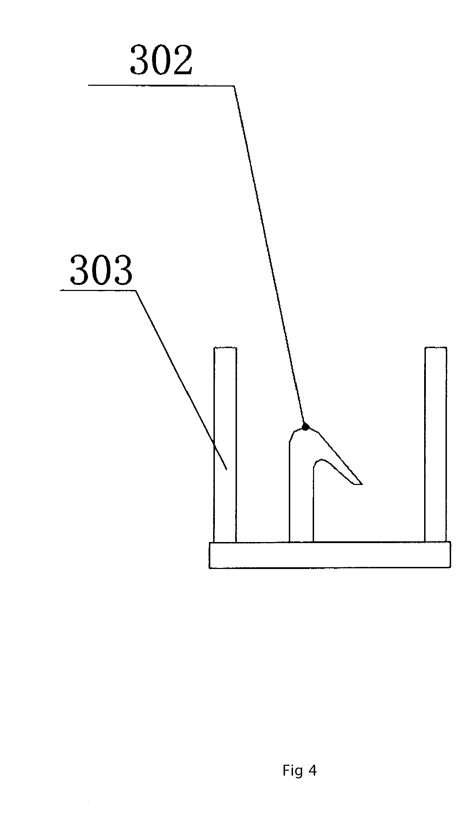

When the invention is carried out in detail as shown in fig.4, the safety sheath 3 is provided therein with two needle tube hooks 302 which has a hook body root which is provided at the bottom of the cavity between the protection wings 303, and which has at the end portion a hook body which has a bending point forming a sharp angle which enables the needle tube safety hook to be in mesh with the needle tube. The two needle tube safety hooks 302 have hook bodies directed in opposite direction so that such opposite double hook structure ensures the needle tube safety hook cannot separate from the needle tube after they are engaged with each other.

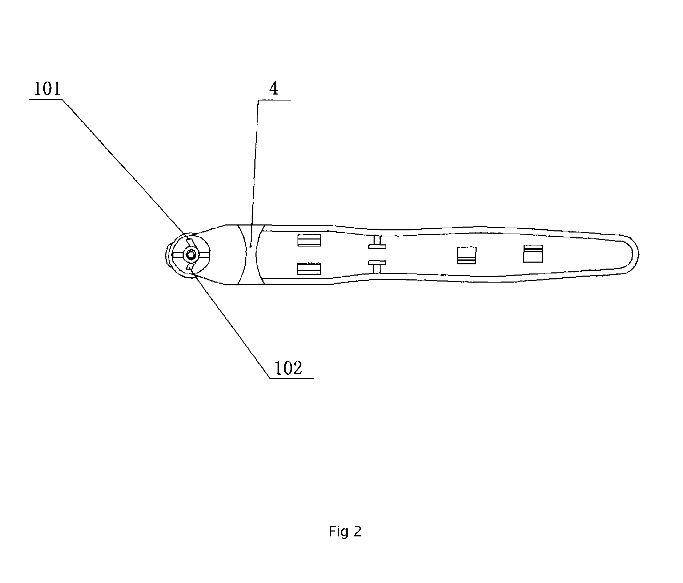

When the invention is carried out in detail as shown in fig.2, the needle base safety hook 101 is in form of two symmetrical reversed toothed plates 102 each of which has a tooth root on the outer wall of the needle base 1 and a tooth end in the air.

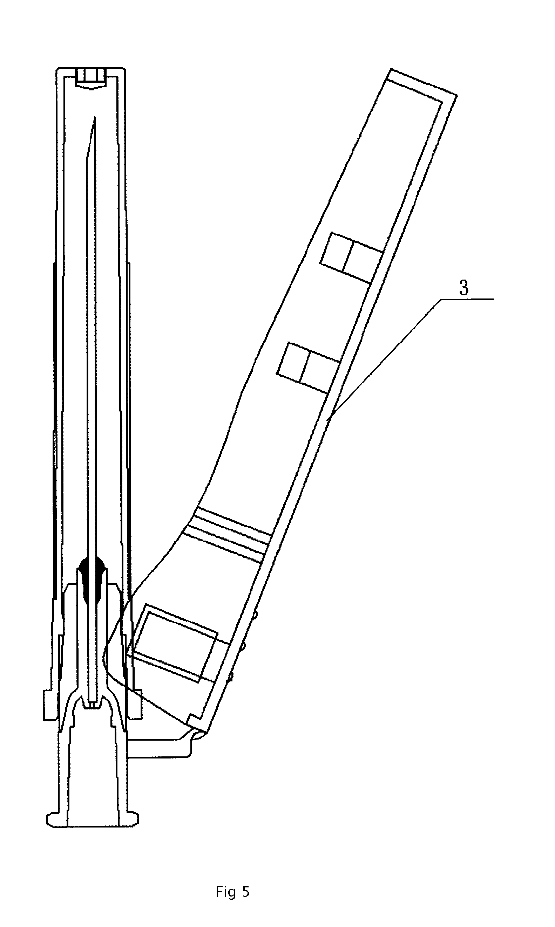

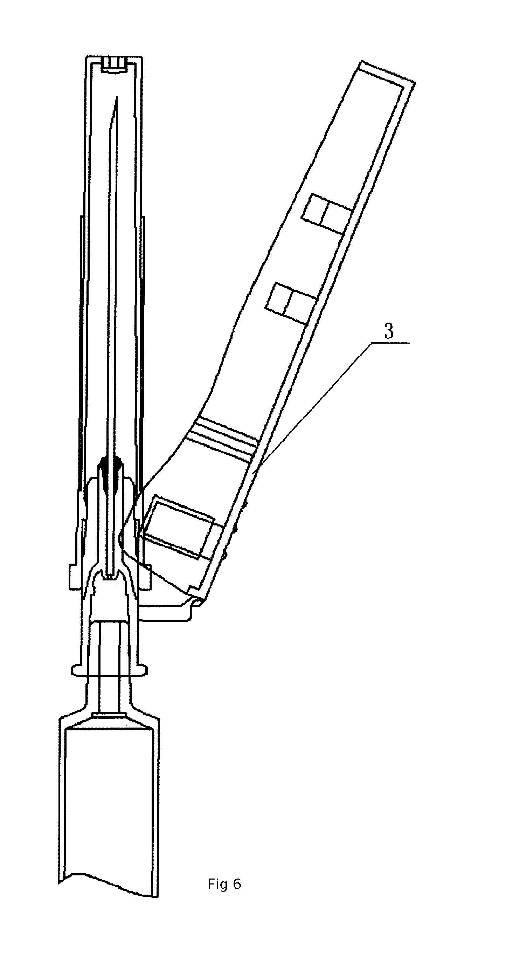

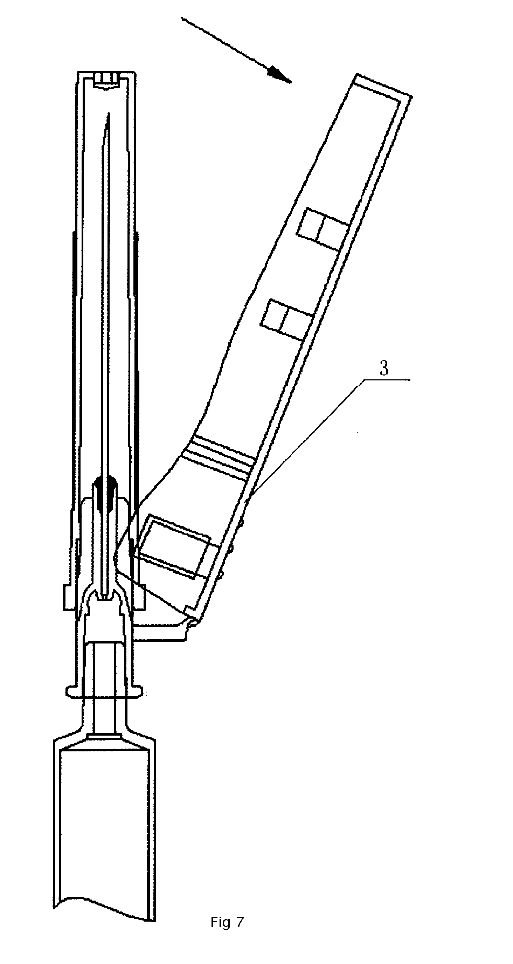

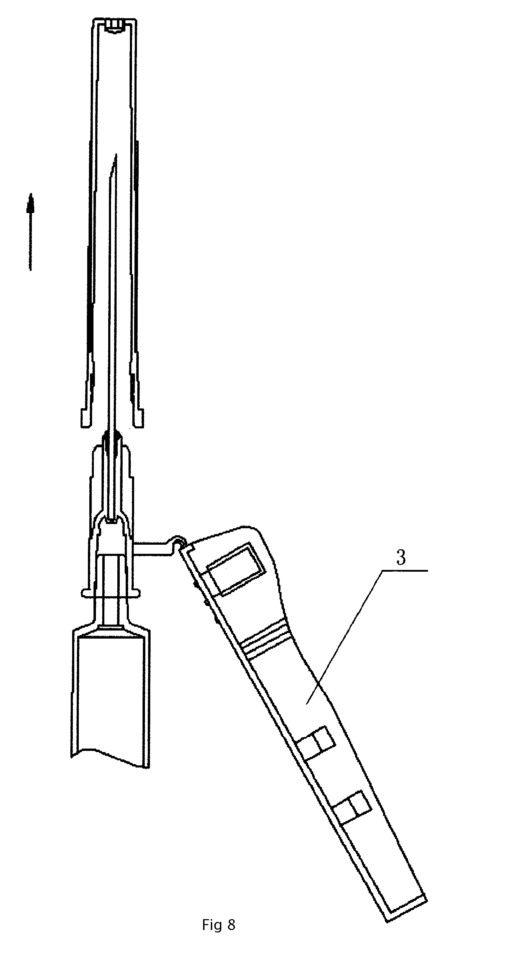

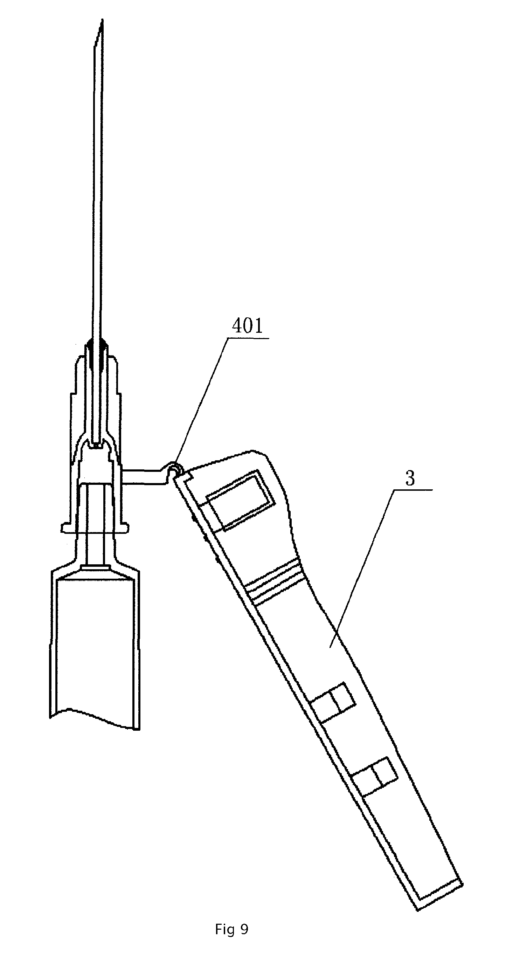

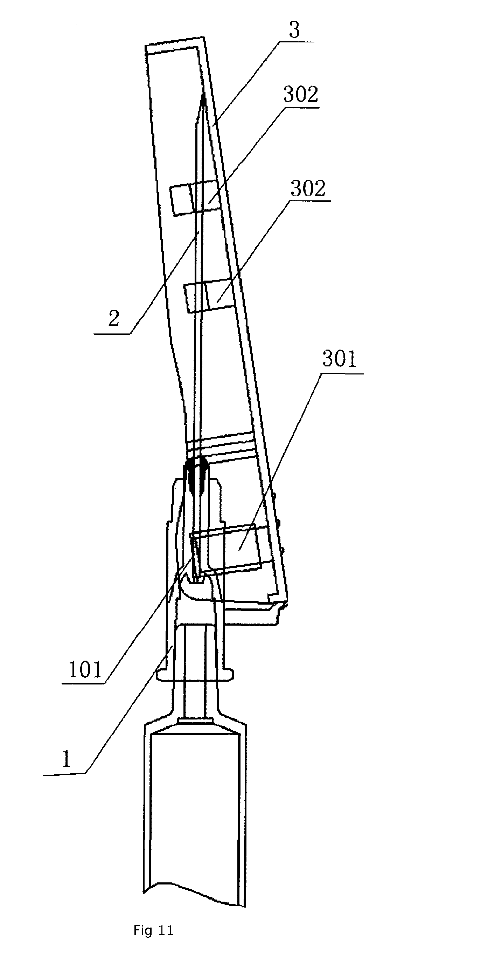

As shown in fig. 5 which shows an initial position of the safety injection needle, in which the safety sheath leans against the sheath automatically under the influence of the elastic telescopic plate so that it is convenient to package the safety injection needle. As shown in fig.6, the needle base of the safety injection needle is put in the conehead of the sterile injection needle; As shown in fig.7, the safety sheath is pushed downwardly, and resumes downwardly automatically under the influence of the elastic telescopic plate, which makes the injection easier to be done. As shown in fig.8, the sheath is removed; As shown in fig.9, the action of injection is taken; As shown in fig. 10, the safety sheath is pushed upwardly after the injection; As shown in fig. 11, the safety sheath is pushed upwardly till the sheath safety hook engages with the needle base safety hook and the needle tube safety hook hooks the needle tube.

The above is a descriptions in detail of the present invention, in connection with the detailed preferably embodiments, which cannot be deemed to limit the scope of the invention by the whole embodiments of the present invention. With respect to the present invention, those skilled in the art, without being away from the principle of the present invention, can devise some solutions by means of deduction and/or replacement, which should also be regarded as protective scope of the present invention.

## Claims

1. A safety injection needle, comprising a needle base (1) provided on the end portion thereof with a needle tube (2), **characterized in that** the needle base (1) being provided at one side with a safety sheath (3), an elastic telescopic plate (4) being provided at the junction between the safety sheath (3) and the needle base (1), the safety sheath 3 rotatable about the elastic telescopic plate (4) which can be seen as a central supporting point,
the safety sheath (3) having a hollow cavity which is provided therein in sequence with a sheath safety hook (301) which is located adjacently to the elastic telescopic plate (4) and a needle tube safety hook (302), the needle base (1) being provided with a needle base safety hook (101) cooperating with the sheath safety hook (301).

2. A safety injection needle as claimed in claim1, characterize in that the needle base (1) being provided with a sheath (5) on the outer portion thereof.

3. A safety injection needle as claimed in claim1, characterize in that the safety sheath (3) being ringent on the surface thereof, provided on two sides with protective wings (303), the sheath safety hook (301) being formed by two symmetrical reversed hook teeth each of which has a tooth root on the inner wall of the protective wings, and a tooth ends in the air.

4. A safety injection needle as claimed in claim1, characterize in that the safety sheath (3) being provided therein with two needle tube hooks (302) each of which has a hook body root at bottom portion of the cavity between the protective wings, and at the end portion a hook body, having a bending point forming a sharp angle, the hook bodies of the two needle tube safety hook being directed in opposite direction.

5. A safety injection needle as claimed in claim1, characterize in that the needle base safety hook (101) being in form of two symmetrical reversed toothed plates (102),each of which has a tooth root on the outer wall of the needle base (1), and a tooth end (2) in the air.

6. A safety injection needle as claimed in claim1, characterize in that an arc upheaval (401) being formed at the elastic telescopic plate.
